(19) **Europäisches Patentamt / European Patent Office / Office européen des brevets**

(11) **EP 4 253 957 A1**

(12) **EUROPEAN PATENT APPLICATION**
published in accordance with Art. 153(4) EPC

(43) Date of publication:
**04.10.2023 Bulletin 2023/40**

(21) Application number: **21898501.8**

(22) Date of filing: **18.11.2021**

(51) International Patent Classification (IPC):
*G01N 33/574* (2006.01)    *G16B 15/30* (2019.01)
*G16B 40/20* (2019.01)    *G06N 3/08* (2023.01)

(52) Cooperative Patent Classification (CPC):
**G01N 33/574; G06N 3/08; G16B 15/30;
G16B 40/20**

(86) International application number:
**PCT/KR2021/017030**

(87) International publication number:
**WO 2022/114675 (02.06.2022 Gazette 2022/22)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.11.2020 KR 20200159098
12.11.2021 KR 20210156076**

(71) Applicant: **Korea Institute of Science and
Technology
Seoul 02792 (KR)**

(72) Inventors:
• **LEE, Kwan Hyi
Seoul 02792 (KR)**

• **KIM, Ho Jun
Seoul 02792 (KR)**
• **JEONG, Youngdo
Seoul 02792 (KR)**
• **PARK, Sungwook
Seoul 02792 (KR)**

(74) Representative: **Longland, Emma Louise
Venner Shipley LLP
Byron House
Cambridge Business Park
Cowley Road
Cambridge CB4 0WZ (GB)**

Remarks:
The complete document including Reference
Table(s) and the Sequence Listing(s) can be
downloaded from the EPO website

(54) **BIOMARKERS FOR DIAGNOSING PROSTATE CANCER, COMBINATION THEREOF, AND USE
THEREOF**

(57)    The present disclosure relates to a biomarker for diagnosis of prostate cancer and use of the biomarker.

According to a biomarker composition for diagnosis of prostate cancer of the present disclosure, an optimal biomarker combination capable of effectively diagnosing prostate cancer has been discovered, and there is an advantage in that prostate cancer can be diagnosed with high accuracy by using the biomarker combination and a machine learning algorithm model.

FIG. 1A

**Description**

Technical Field

The present disclosure relates to a biomarker for diagnosis of prostate cancer and use of the biomarker.

Background Art

Prostate cancer is a malignant tumor that occurs in the prostate in males, and is known as a high-risk disease with high incidence. In particular, prostate cancer is known as the second most common cause of cancer death in the USA. Furthermore, according to the National Cancer Registration and Statistics of 2016, the number of new cancer patients in 2016 was reported to have increased by 12,638 (5.8 %) from 212,542 in the previous year (in 2015) to 229,180 (male: 120,068, female: 109,112). Among these statistics, as compared to 2015, prostate cancer was reported as the fourth most common cancer, surpassing liver cancer.

Likewise, as Korean society has recently become an aging society, the prevalence rate of prostate cancer seems to have increased. The reason for the increase is that, considering that the most important risk factor for prostate cancer is age, prostate cancer occurs mostly in elderly people over the age of 60. However, in reality, prostate cancer frequently develops even in males in their 50s or younger. Therefore, prostate cancer is regarded as a chronic disease requiring constant management, and in this regard, it is time for an accurate diagnosis of prostate cancer. Also, since prostate cancer shows an unpredictable disease progression form, prediction in advance through prognosis is necessary.

Meanwhile, as of now, methods of cancer diagnosis have been performed in an invasive manner, such as tissue sample collection and endoscopy. In particular, a biopsy has been performed by extracting a portion of an area where disease is suspected and observing the extracted portion under a microscope. Thus, when a needle or punch is used or an endoscope or laparoscope is carried out to collect a tissue sample, the human body must be incised. Not only does the patient experience considerable discomfort, but they are also left with a scar and require a long period of time for recovery. As the conventional techniques for diagnosis of prostate cancer, prostate-specific antigen (PSA) measurement and biopsies have been used. The PSA measurement is the most common method for diagnosis of prostate cancer, and is a way of determining the risk of prostate cancer by measuring a level of a specific antigen produced by prostate cells. PSA levels are higher with more malignant prostate cancer, and most males without prostate cancer have a PSA level of less than 4 ng/mL. However, when the measured PSA levels are significantly high even in the absence of prostate cancer, or are in a PSA gray zone range (PSA 4 ng/mL or more and 10 ng/mL or less), a biopsy is required. A biopsy is a method of diagnosing the presence or absence of malignant tumors, such as cancer or sarcoma, in an organ suspected of cancer in a patient through histopathological examination after tissue of the organ is obtained using a needle. However, a biopsy test is very painful for patients, and in particular, accuracy of diagnosis of prostate cancer in the PSA gray zone is less than 30 %. In other words, due to the limitations of PSA, about 70 % of patients suffer from re-biopsy that causes severe pain and economic burden.

Therefore, to overcome the limitations described above, a combination of multiple prostate cancer-specific biomarkers for diagnosis of prostate cancer was discovered in the present disclosure. In addition, correlation between the measurement signals of multiple biomarkers and prostate cancer was analyzed by using an artificial intelligence technique, thereby completing the present disclosure.

Disclosure

Technical Problem

An aspect provides a biomarker composition for diagnosis of prostate cancer, the biomarker composition including one or more selected from the group consisting of ANXA3 (annexin A3), PSMA (prostate-specific membrane antigen), ERG (erythroblast transformation-specific related gene protein), and ENG (endoglin).

Another aspect provides a composition for diagnosis of prostate cancer, the composition including an agent capable of measuring an expression level of one or more proteins selected from the group consisting of ANXA3 (annexin A3), PSMA (prostate-specific membrane antigen), ERG (erythroblast transformation-specific related gene protein), and ENG (endoglin), or genes encoding the same.

Another aspect provides a kit for diagnosis of prostate cancer, the kit including the composition for diagnosis of prostate cancer.

Another aspect provides a method of providing information on prostate cancer diagnosis, the method including measuring an expression level of one or more proteins selected from the group consisting of ANXA3 (annexin A3), PSMA (prostate-specific membrane antigen), ERG (erythroblast transformation-specific related gene protein), and ENG (endoglin), or genes encoding the same, in a biological sample isolated from a subject.

Technical Solution

An aspect provides a biomarker composition for diagnosis of prostate cancer, the biomarker composition including one or more selected from the group consisting of ANXA3 (annexin A3), PSMA (prostate-specific membrane antigen), ERG (erythroblast transformation-specific related gene protein), and ENG (endoglin).

The biomarker composition may diagnose, in a specific subject, prostate cancer and/or predict prognosis of prostate cancer, by detecting or measuring an expression level of one or more proteins selected from the group consisting of ANXA3, PSMA, ERG, and ENG, or genes encoding the same.

The term "diagnosis" as used herein refers to identification of the presence or properties of pathological states. Regarding the purpose of the present disclosure, the diagnosis may refer to determination of the development of prostate cancer.

The term "prognosis" as used herein refers to prediction of disease progression and recovery, and also refers to a prospect or a preliminary evaluation. Regarding the purpose of the present disclosure, the diagnosis may refer to determination of success of treatment, survival, recurrence, metastasis, drug response, resistance, and the like, in a corresponding subject after treatment of prostate cancer. That is, the prognosis means the prediction of medical consequences (for example, organ viability, disease-free survival rates, and the like), and may include positive prognosis or negative prognosis. The negative prognosis includes disease progression or mortality in terms of recurrence, tumor growth, metastasis, drug resistance, or the like, and the positive prognosis includes remission of disease in a disease-free state or the like, or improvement or stabilization of a disease such as tumor regression.

The term "prediction" as used herein refers to assumption in advance for medical consequences. Regarding the purpose of the present disclosure, the prediction may mean assuming in advance the progress of a disease (for example, disease progression, improvement, cancer recurrence, tumor growth, drug resistance, or the like) of a patient diagnosed with prostate cancer.

The term "prostate cancer (PCa)" as used herein refers to cancer occurring in the prostate. The prostate is a male reproductive organ about the size of a walnut located right below the bladder and in front of the rectum, and is responsible for producing and saving a part of semen. The prostate is adjacent to the bladder neck upward, i.e., an area from the bladder to the urethra, so that the prostate is fixed with the ligament puboprostaticum frontward and is also fixed with the urogenital diaphragm downward. Most of cancers occurring in the prostate are adenocarcinoma (cancer of glandular cells) that occurs in prostate cells, and cancer types can be classified according to the degree of differentiation of a tumor tissue and cell characteristics.

The term "biomarker" as used herein generally refers to an indicator that can sense a change in the body by using an organic biomolecule, such as a protein, a nucleic acid (for example, DNA and mRNA), a metabolite (for example, lipid, glycolipid, glycoprotein, and sugar). In detail, the biomarker may refer to an indicator that can distinguish normal conditions from pathological conditions in case of a specific disease or cancer, or that can predict and objectively measure a therapeutic response. Depending on usage, the biomarker may include a target marker that identifies the presence of a drug target, a diagnostic marker for diagnosing the presence or absence of disease, a predictive marker that can distinguish a responder group and a non-responder group with respect to a specific drug, a surrogate marker that can monitor a drug therapeutic effect, a prognostic biomarker that inform the prognosis of disease, and the like.

The ANXA3 (annexin A3) is a protein encoded by the *ANXA3* gene, and belongs to the annexin family. This protein is known to have a function of forming inositol 1-phosphate by inhibiting phospholipase A2 and cleaving inositol 1,2-cyclic phosphate, and may also have an anticoagulant action. The ANXA3 may have an amino acid sequence of Uniprot Accession No. P12429 in humans.

The ANXA3 may preferably have an amino acid sequence of SEQ ID NO: 1, but may have a sequence that is 90 % or more, 93 % or more, 95 % or more, 96 % or more, 97 % or more, 98 % or more, or 99 % or more identical to the amino acid sequence of SEQ ID NO: 1.

The PSMA (prostate-specific membrane antigen) is a protein encoded by the *FOLH1* gene, and is also known as glutamate carboxypeptidase II (GCPII). The PSMA is known to be expressed mainly in the prostate epithelium, the proximal tubule of the kidney, the brush border of the jejunum in the small intestine, and the ganglion of the nervous system. In particular, human PSMA is highly expressed in the prostate, and in some prostate cancer cases, PSMA is expressed at an 8-12 fold higher level than in noncancerous prostate cells. The PSMA may have an amino acid sequence of Uniprot Accession No. Q04609 in humans.

The PSMA may preferably have an amino acid sequence of SEQ ID NO: 2, but may have a sequence that is 90 % or more, 93 % or more, 95 % or more, 96 % or more, 97 % or more, 98 % or more, or 99 % or more identical to the amino acid sequence of SEQ ID NO: 2.

The ERG (erythroblast transformation-specific related gene protein) is a protein encoded by the *ERG* gene, and is known to serve as a transcriptional regulator. The ERG is expressed at a higher level in early myelocytes than in mature lymphocytes, and thus the ERG may serve as a regulator of differentiation of early hematopoietic cells. In addition, the *ERG* gene is classified as a proto-oncogene, and may be fused with a different chromosome during chromosomal translocations that occur in cell division. The ERG may have an amino acid sequence of Uniprot Accession No. P11308 in humans.

The EFG may preferably have an amino acid sequence of SEQ ID NO: 3, but may have a sequence that is 90 % or more, 93 % or more, 95 % or more, 96 % or more, 97 % or more, 98 % or more, or 99 % or more identical to the amino acid sequence of SEQ ID NO: 3.

The ENG (endoglin) is a protein encoded by the ENG gene, and is a type I membrane glycoprotein located on the surface of cells. The ENG constitutes a part of a TGF beta receptor complex. The ENG plays a vital role in angiogenesis, and is known as a protein important for tumor growth, survival, and metastasis of cancer cells in various areas of the body. The ENG may have an amino acid sequence of Uniprot Accession No. P17813 in humans.

The ENG may preferably have an amino acid sequence of SEQ ID NO: 4, but may have a sequence that is 90 % or more, 93 % or more, 95 % or more, 96 % or more, 97 % or more, 98 % or more, or 99 % or more identical to the amino acid sequence of SEQ ID NO: 4.

The biomarker for diagnosis of prostate cancer may include a combination of two or more selected from the group consisting of ANXA3, PSMA, ERG, and ENG, particularly, a biomarker combination selected from PSMA/ERG, PSMA/ENG, ENG/ERG, ANXA3/ENG/ERG, PSMA/ENG/ERG, and ANXA3/PSMA/ENG/ERG, and more particularly, a biomarker combination of ERG and ENG. In addition, the biomarker for diagnosis of prostate cancer may not include ANXA3 to improve diagnostic accuracy.

Another aspect provides a composition for diagnosis of prostate cancer, the composition including an agent capable of measuring an expression level of one or more proteins selected from the group consisting of ANXA3, PSMA, ERG, and ENG, or genes encoding the same. The same contents as described above also apply to the descriptions of the composition.

The term "measuring an expression level" as used herein refers to measuring the presence, occurrence of expression, or extent of expression of a specific protein (peptide) or a gene encoding the protein. In detail, the term may refer to measuring an expression level of one or more proteins selected from the group consisting of ANXA3, PSMA, ERG, and ENG, or mRNA or genes encoding the same.

The composition for diagnosis of prostate cancer may include an agent(s) capable of measuring an expression level of two or more proteins selected from the group consisting of ANXA3, PSMA, ERG, and ENG, or mRNA or genes encoding the same. Particularly, the composition may include an agent(s) capable of measuring an expression level of a biomarker combination selected from PSMA/ERG, PSMA/ENG, ENG/ERG, ANXA3/ENG/ERG, PSMA/ENG/ERG, and ANXA3/PSMA/ENG/ERG, and more particularly, an agent(s) capable of measuring an expression level of ERG and ENG proteins or mRNA or genes encoding the proteins. In addition, the composition for diagnosis of prostate cancer may not include an agent capable of measuring an expression level of ANXA3 or mRNA or gene encoding the same, to improve diagnosis accuracy.

A method of measuring the expression level of the protein may include Western blotting, enzyme linked immunosorbent assay (ELISA), radioimmunoassay (RIA), radical immunodiffusion, Ouchterlony immunodiffusion, rocket immunoeletrophoresis, immunohistochemical staining, immunoprecipition assay, complement fixation assay, immunofluorescence, immunochromatography, fluorescenceactivated cell sorter analysis (FACS), protein chip technology, or biosensor.

A method of measuring the expression level of the mRNA or genes may include reverse transcriptase polymerase chain reaction (RT-PCR), competitive RT-PCR, real time quantitative RT-PCR, quantitative RT-PCR, a RNase protection method, Northern blotting, DNA chip technology, or a biosensor.

The terms "agent capable of measuring an expression level" as used herein refers to a molecule available to confirm an expression level of a specific protein or a gene encoding the same. Particularly, the agent may include a material capable of detecting and/or amplifying the protein or the gene encoding the same, but is not limited thereto.

The term "agent capable of detecting a specific protein or a gene encoding the same" as used herein refers to a material allowing specific binding the specific gene or the protein to be recognized, or a material capable of sensing and amplifying the specific gene or protein. The term "agent capable of amplifying a specific gene or a protein" as used herein refers to a material capable of increasing the number of the specific gene or the protein by repeating replication thereof. For example, the agent may be a primer capable of specifically amplifying a polynucleotide including the gene, or a probe capable of specifically binding to the polynucleotide, but is not limited thereto.

The composition for diagnosis of prostate cancer may include a primer, a probe, a nucleotide, an antibody, an antibody fragment or an antigen-binding fragment thereof, a ligand, a receptor, a protein, or a combination thereof, each specifically binding to one or more proteins selected from the group consisting of ANXA3, PSMA, ERG, and ENG, or mRNA or genes encoding the same.

The term "primer" as used herein refers to a nucleic acid sequence having a free 3'-hydroxyl group, capable of forming a base pair with a template complementary to a specific base sequence, and serving as a starting point for replication of a template strand. The primer may be able to initiate DNA synthesis in the presence of a reagent (i.e., a DNA polymerase or a reverse transcriptase) and four different nucleoside triphosphates, for polymerization using an appropriate buffer at an appropriate temperature. For example, as a primer specific to a gene or mRNA encoding one or more proteins selected from the group consisting of ANXA3, PSMA, ERG, and ENG, sense and antisense primers having a sequence of 7 to 50 nucleotides are used for PCR amplification. By measuring an amount of a desired product, a subject can be diagnosed whether the subject develops prostate cancer. PCR conditions and lengths of sense and antisense primers may be appropriately selected according to techniques known in the art. The primer may have a length between 10 nt to 100 nt, 15 nt to 100 nt, 10 nt to 80 nt, 10 nt to 50 nt, 10 nt to 30 nt, 10 nt to 20 nt, 15 nt to 80 nt, 15 nt to 50 nt, 15 nt to 30 nt, 15 nt to 20 nt, 20 nt to 100 nt, 20 nt to 80 nt, 20 nt to 50 nt, or 20 nt to 30 nt.

The term "probe" as used herein refers to a nucleic acid fragment, such as RNA or DNA, that can specifically bind to a target nucleic acid, for example, mRNA. Here, the probe may be labeled to determine the presence or absence, amount, and expression level of specific mRNA. The probe may be prepared in the form of an oligonucleotide probe, a single-stranded DNA probe, a double-stranded DNA probe, an RNA probe, or the like. For example, a probe having a nucleic acid sequence complementary to a gene or mRNA encoding one or more proteins selected from the group consisting of ANXA3, PSMA, ERG, and ENG may be used for hybridization. By measuring an expression level of mRNA through the degree of hybridization, a subject can be diagnosed whether the subject develops prostate cancer. Selection of suitable probe and hybridization conditions may be appropriately selected according to techniques known in the art. The probe may have a length between 10 nt to 100 nt, 15 nt to 100 nt, 10 nt to 80 nt, 10 nt to 50 nt, 10 nt to 30 nt, 10 nt to 20 nt, 15 nt to 80 nt, 15 nt to 50 nt, 15 nt to 30 nt, 15 nt to 20 nt, 20 nt to 100 nt, 20 nt to 80 nt, 20 nt to 50 nt, or 20 nt to 30 nt.

The primer or probe may be chemically synthesized by using a phosphoramidite solid support synthesis method or other well-known methods in the art. In addition, such a nucleic acid sequence may be modified through various methods known in the art. An example of modifications includes methylation, encapsulation, substitution with one or more homologues of natural nucleotides, or modification between nucleotides, such as modification to uncharged linkages (e.g., methyl phosphonate, phosphotriester, phosphoroamidate, carbamate, or the like) or to charged linkages (e.g., phosphorothioate, phosphorodithioate, or the like). Also, the primer or probe may be modified with a label that can provide a detectable signal in a direct or indirect manner. An example of the label includes a radioactive isotope, a fluorescent molecule, or a biotin.

The term "antibody" as used herein is a term known in the art, and refers to a specific immunoglobulin directed against an antigenic site. For example, the antibody may specifically bind to one or more proteins or fragments thereof selected from the group consisting of ANXA3, PSMA, ERG, and ENG. The fragment refer to a protein fragment having one or more epitopes that can be recognized by an antibody against the protein, and may be, for example, an immunogenic fragment. The form of the antibody may include a polyclonal antibody, a monoclonal antibody, or a recombinant antibody, and may include all immunoglobulin antibodies. In addition, the antibody may include a specialized antibody, such as a humanized antibody.

By using these antibodies, ELISA (enzyme linked immunosorbent assay), RIA (radioimmunoassay), sandwich assay, or Western blotting or immunoblotting on polyacrylic gels known in the art may be performed to confirm whether the protein is expressed in a biological sample.

The term "antibody fragment" as used herein refers to a polypeptide that does not have the structure of an intact antibody, peptide or protein, but has a specific antigen-binding site or binding domain directed against the antigenic site. The fragment may include a functional fragment of an antibody molecule rather than an intact antibody having two light chains and two heavy chains. The functional fragment of the antibody molecule refers to a fragment having at least an antigen-binding function, and may be Fab, F(ab'), F(ab')$_2$, or Fv. The binding fragment may include at least 7 amino acids, for example, 9 or more amino acids or 12 or more amino acids.

Another aspect provides a kit for diagnosis of prostate cancer, the kit including the composition for diagnosis of prostate cancer. The same contents as described above also apply to the descriptions of the kit.

The kit may sense a biomarker by detecting expression or measuring an expression level of one or more proteins selected from the group consisting of ANXA3, PSMA, ERG, and ENG which are biomarkers for diagnosis of prostate cancer, or genes encoding the same.

The kit may include: not only a primer, a probe, or an antibody selectively recognizing a marker, or an antibody fragment retaining antigen-binding ability, for detecting expression or measuring an expression level of one or more proteins selected from the group consisting of ANXA3, PSMA, ERG, and ENG, or genes encoding the same; but also one or more other constituent compositions or apparatuses suitable for the measurement or analysis method.

For example, the kit for diagnosis of prostate cancer to detect or measure an expression level of a polynucleotide or a gene may include one or more oligonucleotides that specifically bind to a polynucleotide or a gene encoding one or more proteins selected from the group consisting of ANXA3, PSMA, ERG, and ENG. Here, the kit may include a primer corresponding to the gene or a partial sequence thereof, a reverse transcriptase, a Taq polymerase, a PCR primer, and dNTP. To measure an expression level of the polynucleotide, the kit using the measurement method described in connection with the expression level of mRNA or gene may be used.

Also, the kit for diagnosis of prostate cancer to detect or measure an expression level of a polypeptide or a protein may include an antibody or a fragment thereof that can specifically bind to one or more proteins or a fragment thereof selected from the group consisting of ANXA3, PSMA, and ERG, and ENG. To measure the expression level of the polypeptide, the kit using the measurement method described in connection with the expression level of the proteins may be used.

The kit may be an electrochemical biosensor or a biosensor kit, for diagnosis of prostate cancer. In detail, the kit may include: an agent(s) for detecting expression or measuring an expression level of one or more proteins selected from the group consisting of ANXA3, PSMA, ERG, and ENG, or genes encoding the same; and a biosensor for measurement and analysis methods.

The term "biosensor" or "bio chip" as used herein refers to a biological microchip that can analyze gene expression patterns, gene defects, protein distribution, response patterns, or the like, by binding a biological molecule such as DNA or a protein to a small substrate formed of glass, silicon, or a nylon material. A biological receptor having a recognition function for a specific material may be linked to an electrical or optical transducer to convert a biological interaction and a recognition response into an electrical or optical signal, so as to selectively sense a substance to be analyzed. Here, the specific material may include not only a biomaterial such as DNA or blood sugar, but also a general chemical substance. The biological receptor refers to a biomolecule that selectively recognizes an analyte and simultaneously generates a signal that a transducer can measure, and examples of the biological receptor are enzymes, proteins, DNA, viruses, cells, hormones, biological membranes, tissues, and the like.

The biosensor for diagnosis of prostate cancer may be operated by an electrical sensing method, which is a method of sensing changes in electrical properties of the sensor induced when a target biomaterial (e.g., DNA, protein, or the like) to be sensed specifically binds to a probe material of a sensor sensing unit. In detail, the biosensor may be a field-effective transistor (FET) biosensor.

Regarding the FET biosensor, a FET-type biosensor using a nanometer thickness channel for connecting a source and a drain through the channel may be operated by a label-free method according to the principle of detecting a change in electrical conductivity inside the channel caused by the charge of the biomaterial itself which binds to the surface of the sensing channel.

In an embodiment of the present disclosure, to detect the biomarker for diagnosis of prostate cancer from a sample (specifically, a urine sample), a dual-gate field-effect transistor (DGFET) biosensor consisting of FET (field-effect transistor) and EG (extended gate) was used. In detail, an agent (specifically, an antibody or a fragment thereof for detecting the biomarker for diagnosis of prostate cancer) is attached to the EG, and the expression level of the biomarker may be measured by measuring an electrical signal generated when the biomarker is bound to the detection agent.

Another aspect provides a method of providing information on prostate cancer diagnosis, the method including measuring an expression level of one or more proteins selected from the group consisting of ANXA3, PSMA, ERG, and ENG, or genes encoding the same, in a biological sample isolated from a subject. The same contents as described above also apply to the descriptions of the method.

The term "subject" as used herein refers to any organism that has developed or is likely to develop prostate cancer, and specific examples thereof are mammals including dogs, cats, mice, rats, monkeys, cattle, pigs, mini-pigs, livestock, humans, and the like, farmed fish, and the like. However, the subject is not limited thereto.

The term "sample" as used herein refers to a material derived from the subject, and examples thereof are tissues, cells, whole blood, serum, plasma, saliva, sputum, cerebrospinal fluid, urine, and the like. However, the sample is not limited thereto. In addition, a gene sample and/or a protein sample may be obtained from the sample, and the gene sample may include a nucleic acid, for example, DNA, mRNA, or cDNA synthesized from mRNA. As long as an expression level of a specific gene/protein can be confirmed from a sample, a sample type is not limited to the above.

The sample may be urine or may be derived from urine. In addition, the sample may be isolated from a subject before or after digital rectal examination (DRE) is performed. In detail, the sample may be isolated from a subject after the DRE is performed.

The method disclosed herein may further include: from a biological sample derived from a control group, measuring an expression level of one or more protein selected from the group consisting of ANXA3, PSMA, ERG, and ENG, or genes encoding the same; and comparing the expression level in the subject with the control group.

The term "control group" as used herein may refer to a general subject who has not developed prostate cancer, a non-prostate cancer patient group, a non-patient group, or the like.

The method disclosed herein may further include determining that the subject has developed prostate cancer or predicting a high level of the risk of developing prostate cancer, when the expression level of one or more proteins selected from the group consisting of ANXA3, PSMA, ERG, and ENG, or genes encoding the same, in the subject is higher than the expression level in the control group.

In addition, the method disclosed herein may refer to measuring an expression level of two or more proteins selected from the group consisting of ANXA3, PSMA, ERG, and ENG, or mRNA or genes encoding the same. Particularly, the method disclosed herein may refer to measuring an expression level of a biomarker combination selected from PSMA/ERG, PSMA/ENG, ENG/ERG, ANXA3/ENG/ERG, PSMA/ENG/ERG, and ANXA3/PSMA/ENG/ERG, and more particularly, may refer to measuring an expression level of ERG and ENG proteins and mRNA or genes encoding the same. In addition, the method disclosed herein may not include measuring an expression level of ANXA3 protein or mRNA or a gene encoding the same, to improve diagnosis accuracy.

The method disclosed herein may further include applying the measured expression level of the proteins or the genes encoding the same to a machine learning algorithm model.

The applying to the machine learning algorithm model may include inputting the expression level of the proteins or the genes encoding the same measured in the subject to the machine learning algorithm model to output, as an output value, whether the subject has developed prostate cancer or is at risk of developing prostate cancer.

An input value entered into the model may be a value obtained by quantifying the expression level of the one or more proteins selected from the group consisting of ANXA3, PSMA, ERG, and ENG, or the genes encoding the same, measured in a subject suspected of being a prostate cancer patient. In detail, the input value may be a voltage shift value measured by using the DGFET biosensor of the present disclosure.

In addition, the output value outputted from the model is a result of determining whether the subject has developed prostate cancer or is at risk of developing prostate cancer. In detail, the output value may be outputted as a predictor value expressed as a number between 0 and 1. When the predictor value is 0.5 or more, the subject may be determined to have developed prostate cancer or be at high risk of developing prostate cancer. When the predictor value is less than 0.5, the subject may be determined to have not developed prostate cancer or be at low risk of developing prostate cancer. In addition, as the predictor value is closer to 0 or 1, the certainty of the algorithm prediction is increased. For example, as the predictor value is closer to 1, the accuracy in predicting the development of prostate cancer in the subject may be increased, and as the predictor value is closer to 0, the accuracy in predicting no development of prostate cancer in the subject may be increased.

The machine learning algorithm model may be learned by setting, as input values, 1) an expression level of one or more proteins selected from the group consisting of ANXA3, PSMA, ERG, and ENG, or genes encoding the same, in a prostate cancer patient group and/or 2) an expression level of one or more proteins selected from the group consisting of ANXA3, PSMA, ERG, and ENG, or genes encoding the same, in a control group. In detail, the input value may be a voltage shift value measured by using the DGFET biosensor of the present disclosure. In addition, a reference voltage shift of the biomarker, such as ANXA3, PSMA, ERG, or ENG, measured by using the DGFET biosensor of the present disclosure may be additionally inputted.

In addition, the machine learning algorithm model may be learned by setting, as an output value, whether prostate cancer is development in the prostate cancer patient group and the control group as previously inputted.

The machine learning algorithm model may include a random forest (RF) algorithm and/or a neural networks (NN) algorithm model.

Advantageous Effects

According to a biomarker composition for diagnosis of prostate cancer of the present disclosure, an optimal combination of biomarkers that can effectively diagnose prostate cancer is discovered. Accordingly, there is an advantage that prostate cancer can be diagnosed with high accuracy by using a biomarker combination and a machine learning algorithm model.

Description of Drawings

FIG. 1 is a diagram schematically describing a biosensor and a system for diagnosis of prostate cancer according to the present disclosure, wherein FIG. 1A shows steps of sensing four biomarkers present in a urine sample by using a DGFET biosensor; and FIG. 1B shows a process of diagnosing prostate cancer through a machine learning-based algorithm based on the biosensor sensing results.

FIGS. 2A to 2D are diagrams showing a linear graph of a reference voltage shift according to biomarker concentration, wherein the reference voltage shift per 100x concentration change is the lowest for ANXA3 (82 mV/dec), and is 145 mV/dec, 129 mV/dec, and 278 mV/dec for PSMA, ERG, and ENG, respectively.

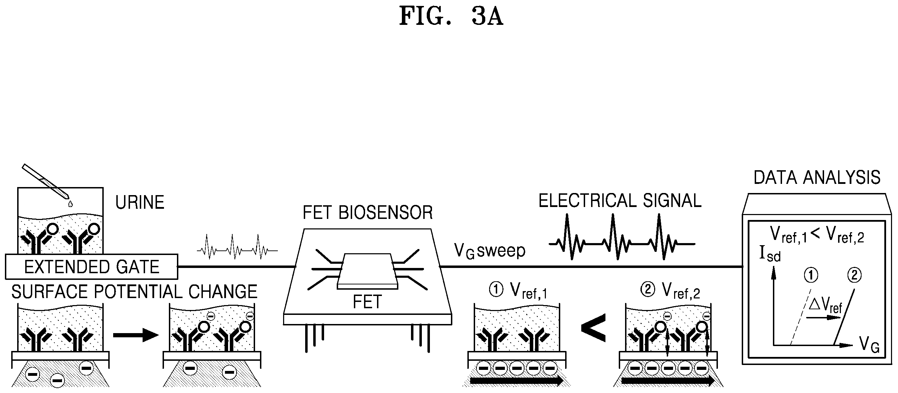

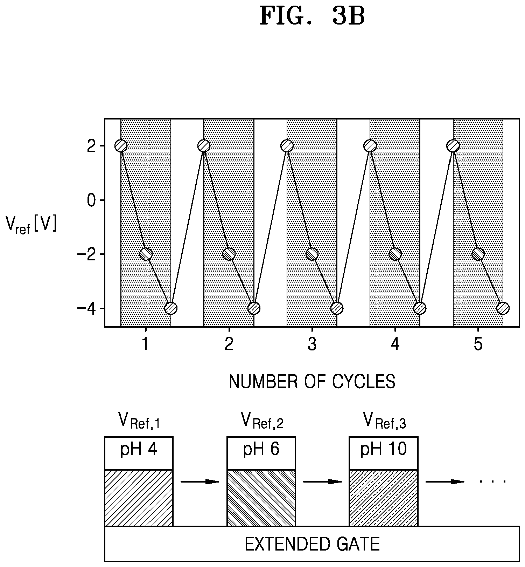

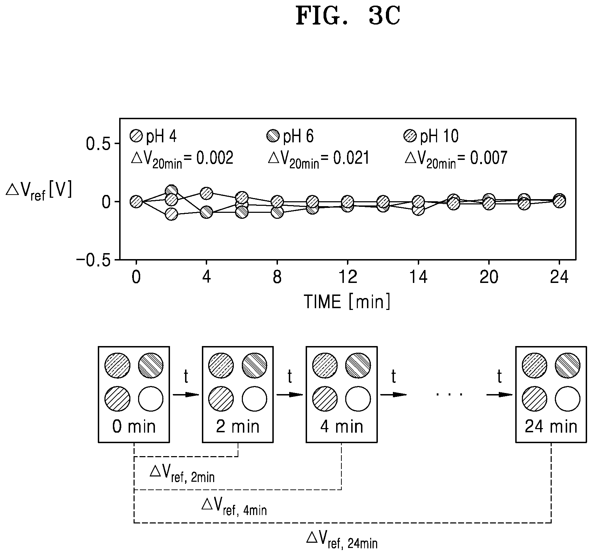

FIG. 3A is a diagram showing a measuring process for a urine sample; FIG. 3B is a diagram showing measurement results for a bottom gate voltage at a reference current (1 nA) after sequentially adding buffer solutions of pH 4, 6, and 10 to the same channel, wherein as a result of repeating measurement 5 times, there is almost no voltage change, showing stable sensing performance in a urine environment; and FIG. 3C is a diagram showing results of measuring a bottom gate voltage for 24 minutes after adding buffers of pH 4, 6, and 10 to each channel, wherein there is almost no voltage change, and a stable sensing performance is exhibited.

FIG. 4 is a diagram showing Gleason score statistics of a sample population.

FIG. 5A is a diagram illustrating a process of diagnosing prostate cancer by using a single biomarker for 76 urine samples; FIG. 5B is a diagram showing results of prostate cancer screening repeatedly performed three times by using each of four biomarkers; and FIG. 5C is a diagram showing results of prostate cancer screening by using each of four biomarkers for each urine sample (***p < 0.001, ****p < 0.0001).

FIG. 6 is a diagram showing results of evaluating sensing performance of the single biomarker in terms of sensitivity, specificity, and accuracy.

FIG. 7 is a diagram illustrating a correlation matrix of a biomarker pair.

FIG. 8 is a diagram describing a process of setting a multi-marker system based on random forest (RF) and neural network (NN) algorithms.

FIG. 9 is a diagram showing results of evaluating sensing characteristics of each biomarker.

FIG. 10 is a diagram showing predictor values by the RF and NN algorithms when various numbers of biomarkers are used.

FIG. 11 is a diagram showing predictor values at a biomarker panel level (FIG. 11A) or at a sample level (FIG.11B).

FIGS. 12A to 12C are diagrams showing results of evaluating diagnosis performance for an additional data set.

FIGS. 13A to 13D are diagrams showing RF algorithm ROC curves of a multimarker sensing system with various numbers of biomarkers at the biomarker panel level.

FIGS. 14A to 14D are diagrams showing NN algorithm ROC curves of a multimarker sensing system with various numbers of biomarkers at the biomarker panel level.

FIG. 15A is a diagram showing the average AUROC values for the various numbers of biomarkers (error bars represent s.d.); and FIG. 15B is a diagram showing AUROC values for the ROC curves of FIGS. 13 and 14.

FIGS. 16A to 16D are each a diagram showing clinical analysis results of error signals predicted by the RF algorithm, specifically, results for the various numbers of biomarker combinations in the test set (total of 69 panels); and FIG. 16E is a diagram showing the number of biomarker panels representing an error rate higher than 0.5.

FIG. 17A is a diagram showing a false positive rate of a biomarker panel for normal and benign prostatic hyperplasia (PBH) patients; and FIG. 17B is a diagram showing a false positive rate of a biomarker panel for groups before and after digital rectal examination (DRE).

FIG. 18 is a diagram showing clinical analysis results in a true positive (TP) group, a true negative (TN) group, a false positive (FP) group, and a false negative (FN) group for four biomarker combinations, specifically in terms of age (FIG. 18A), PSA level (FIG. 18B), and prostate volume (FIG. 18C) (***p < 0.001, ****p < 0.0001).

Mode for Invention

Hereinafter, the present disclosure will be described in more detail with reference to Examples below. However, these Examples are for illustrative purposes only, and the scope of the present disclosure is not intended to be limited by these Examples.

The present disclosure relates to a composition and a method for diagnosis of prostate cancer. In detail, as a specimen (sample) for screening patients who may need a biopsy for diagnosis of prostate cancer, urine was selected. Since the urethra is surrounded by prostatic tissues, biomarkers are passively diffused from prostate cancer (PCa) cells into urine. Thus, urine may be particularly important for PCa screening, and since collection of a urine sample is non-invasive, patient compliance is also improved. However, the concentration of biomarkers is low so that it is difficult to diagnose prostate cancer by using such a urine sample. In the present disclosure, to solve this problem, a high-sensitive dual-gate field-effect transistor (DGFET) was used as a multimarker sensor in urine. The DGFET consists of disposable 4-channel extended gates (EGs) that are separable from the transistor to improve sensing performance and reliability. By using the DGFET biosensor, trace amounts of protein-based biomarkers may be sensed in 20 minutes in patient and animal samples. Also, to extract clinically important information from complicated multimarker sensing signals, two machine learning (ML) algorithms (random forest (RF) and neural network (NN)) were introduced. In particular, by comparing RF with NN, the best algorithms and the best combination of biomarkers that provide the highest accuracy in PCa screening were developed.

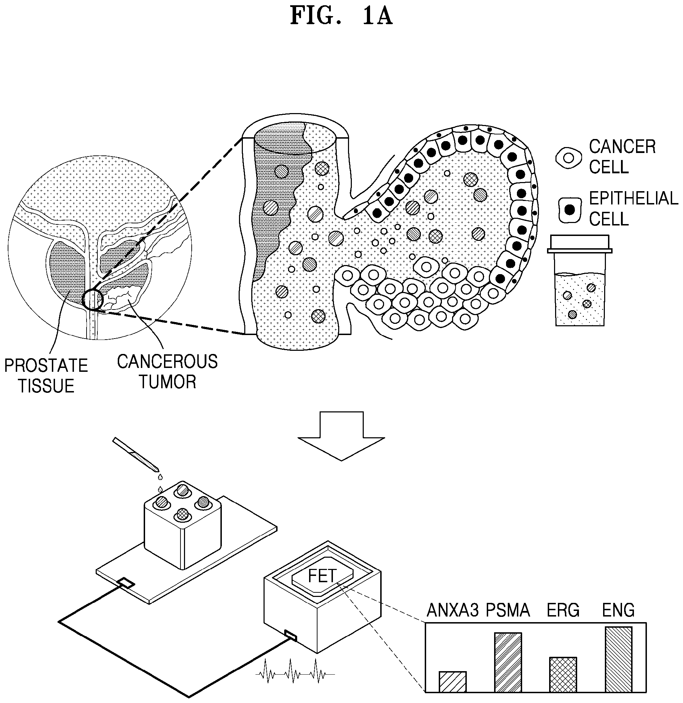

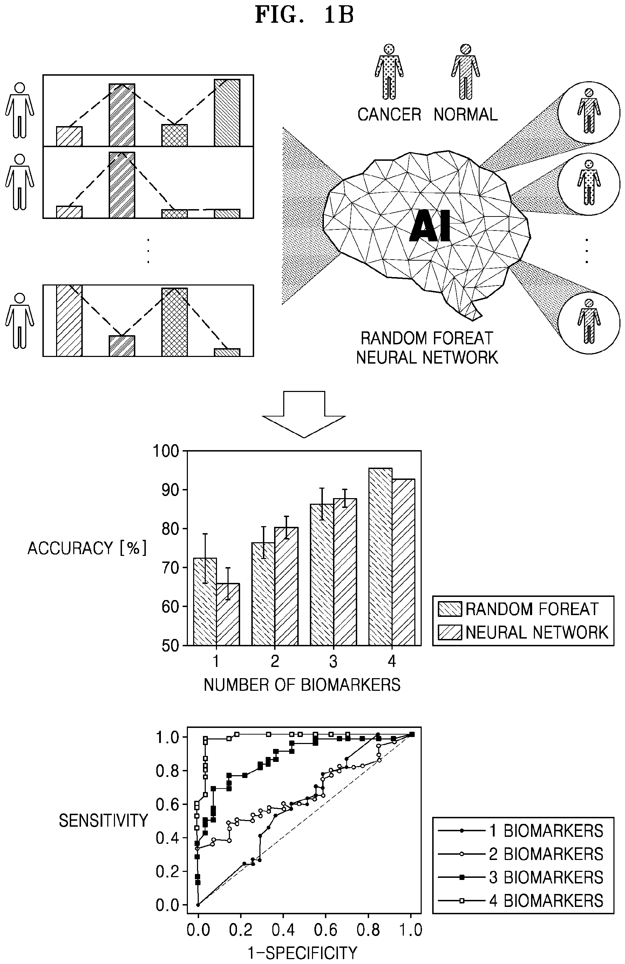

A general overview of the present disclosure illustrated in FIG. 1. In detail, several biomarkers are passively diffused from PCa cells into the urethra, and then transported by urination. Naturally voided urine was collected from potential patients with PCa. Then, such urine samples were applied to a DGFET biosensor to obtain electrical signals from different biomarkers. In the present disclosure, four biomarkers were used: ANXA3 (annexin A3), PSMA (prostate-specific membrane antigen), ERG (erythroblast transformation-specific related gene protein), and ENG (endoglin). Data from 76 urine samples were randomly divided into a training dataset (70 % of the samples) and a test dataset (30 % of the samples). After a training step, the ML algorithm screened PCa patients in the test dataset. The performance of PCa screening may be improved incrementally as more biomarkers are bound. Therefore, use of even a drop of urine was able to represent 100 % accuracy.

Example 1: Manufacture of DGFET biosensor

The DGFET (dual-gate field-effect transistor) consists of a FET (field-effect transistor) and an EG (extended gate), and a DGFET device was manufactured by a method described in the existing document (Adv. Sci. 2019, 6 (11), 1802115). In detail, a 6-inch p-type (100) silicon wafer with a 750 nm-thick-buried oxide layer and a 100 nm-thick-top silicon layer was used. To define an active region, inductively coupled plasma reactive-ion etching (ICP-RIE) and photolithography processes were applied. Then, a 10 nm-thick-oxidation layer was deposited on a top gate according to a dry oxidation process. On this layer, a Ti/TiN/Al/TiN multilayer was grown through ICP-RIE followed by a sputtering system to form a gate electrode. Drain and source parts were formed by implantation of arsenic ions. An EG with four polydimethylsiloxane (PDMS) channels was manufactured on a glass substrate on which an indium tin oxide (ITO, 300 nm) film was formed. The four PDMS channels having a surface area of 0.5 cm$^2$ were prepared by using a hole punch (diameter of 0.8 cm). The four channels were attached to the ITO substrate through plasma bonding after $O_2$ plasma treatment (70 W, 30 sccm of $O_2$ gas flow for 1 minute, Plasma System Cute, Femto Science).

Next, to modify the surface of the EG, hydroxyl groups (-OH) were introduced by $O_2$ plasma treatment at 70 W for 1 minute. Then, amine groups ($-NH_2$) were introduced by dipping the surface in 5 % 3-aminopropyltriethoxysilane (APTES) (ethanol) for 1 hour. After washing with a sonicator, an APTES layer thus obtained was cured at 120 °C for 30 minutes. To conjugate the APTES layer with an antibody or a protein through an amide bond, a 2.5 % glutaraldehyde solution was treated thereon. Afterwards, 5 $\mu$g/ml of antibodies that can sense the four biomarkers (i.e., PSMA, ANXA3, ENG, and ERG) was added to each channel and reacted for 1 hour. After washing with phosphatebuffered saline (PBS), 0.2 M ethanolamine was added for 1 hour to inactivate the unreacted carboxyl acid groups. Then, the EG was cultured with a PBS solution containing 3 % BSA for 1 hour to block nonspecific binding from urine biomolecules.

Example 2: Measurement setup of DGFET biosensor

A dual-channel parameter analyzer (4200A-SCS, Keithley) was used to obtain an $I_D$-$V_G$ curve on the DGFET. In the case of the $I_D$-$V_G$ curve, a sweeping voltage of the bottom gate was between -10 V and 10 V, and a drain voltage was 3 V. To obtain a standard curve, voltage shift at various concentrations of PSMA, ANXA3, ENG, and ERG (0.1 fg/ml to 10 ng/ml) in normal urine was measured. Here, an initial voltage signal was obtained from an $I_D$-$V_{BG}$ curve in a 1x PBS solution. The solution was then removed, and the urine samples were injected directly into the surface-modified EG and reacted for 20 minutes. After washing with a 1x PBS solution three times, the washed solution was removed from the channels. Finally, a clean 1x PBS solution was added and the final voltage signal was measured. The sensing voltage (voltage shift) was obtained by subtracting the initial voltage signal (t = 0 minute) from the final voltage signal (t = 20 minutes) at the reference current (1 nA).

In the case of multimarker sensing, the same procedure as described above was performed, except that the four channels of the EG were conjugated with different antibodies. The initial voltage signal was recorded in a 1X PBS solution, and subsequently, the urine samples were added to each channel and cultured simultaneously for 20 minutes. Then, a washing process was performed thereon three times using a 1x PBS solution. Finally, a clean 1x PBS solution was added and the final voltage signal was measured.

Example 3: Analysis method using artificial intelligence model

To generate predictor values for each biomarker panel, a customized ML software was developed by Python 3.6.1 with multiple open libraries including Scikit Learn, Panda, Numpy, Matplotlib. In the present disclosure, RF decision trees and feedforward neural networks were used for the NN algorithm. RF consists of 80 random decision trees with binary classifications for cancer and normal cases, and majorityvoted results are collected for all biomarker panels. In the case of NN, a feedfoward neural network with three hidden layers of three nodes was used. The NN model was implemented by using Keras with a TensorFlow framework. To train these two algorithms, a supervised learning method was used, wherein the algorithms were repeatedly trained by randomly assigning 70 % of the total dataset. The rest of the blind testset (30 % of total) was then used to validate the screening performance of the algorithms. To evalute the signal combinations and generate the predictor values, the voltage shifts from the four biomarker sensing channels were input into both RF and NN algorithms. When calculating the accuracy, the predictor value of 0.5 was used as a threshold value ($\geq$0.5 for cancer, <0.5 for normal). Each urine sample was measured three times, and an average of the measured predictor values was used to conclude a final decision on the PCa screening.

Example 4: Statistical analysis method

The data provided in Experimental Examples of the present disclosure were obtained from three independent experiments, and unless otherwise indicated, the data were expressed as the mean$\pm$standard deviation. Statistical

comparisons between two groups were performed based on unpaired Student's t-tests. A difference in the case of P<0.05 was considered significant.

Experimental Example 1: Selection of biomarkers for diagnosis of prostate cancer

An experiment was performed as follows to select biomarkers for use in a composition and method for diagnosis of prostate cancer of the present disclosure.

In detail, in consideration of clinical relation to prostate cancer, four pathophysiologically uncorrelated biomarkers that are upregulated in patients with PCa were selected: ANXA3, PSMA, ERG, and ENG. ANXA3 is found in urine with high specificity to PCa, and ANXA3 expression is observed in prostatic intraepithelial neoplasia which is a precursor to PCa. PSMA is a membrane protein expressed on the apical surface of endothelial cells. PSMA overexpression is related to a higher PCa grade and androgen deprivation, suggesting that PSMA plays a functional role in PCa progression. ERG expression is strongly related to an increased level of TMPRSS2/ERG fusion genes that promote tumor progression and invasion. Increased ENG expression is found in prostate endothelial cells during tumor angiogenesis and inflammatory processes. ENG is also expressed in prostate intraepithelial neoplasia, showing potential as a prognostic marker.

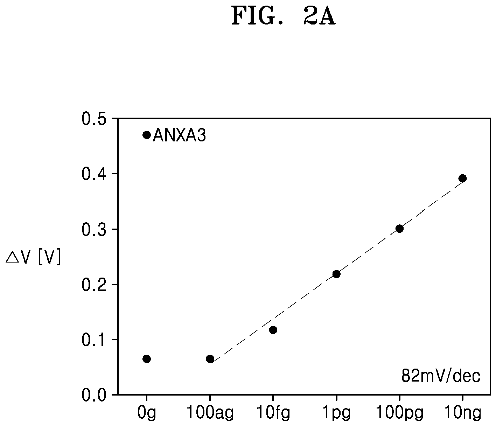

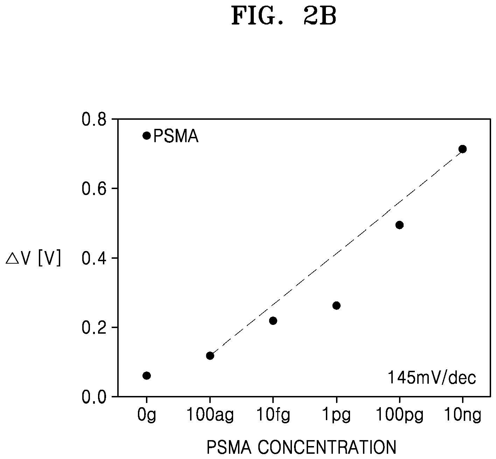

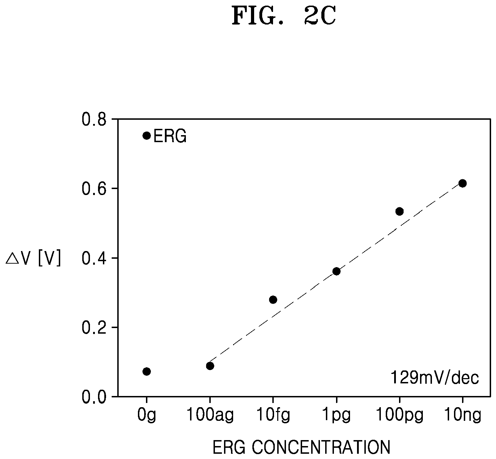

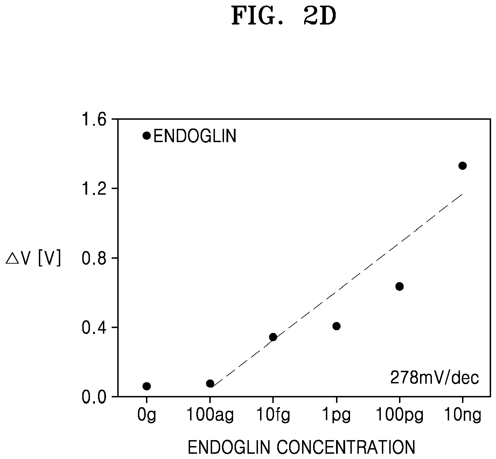

Next, to confirm whether or not the selected four can induce charge polarization and subsequent threshold voltage change in the DGFET system, detailed sensing characteristics were evaluated. As a result, it was confirmed that all of the four biomarkers candidates showed a linear relationship between voltage shifts and concentration levels (see FIGS. 2A to 2D), suggesting that these biomarkers are suitable for the DGFET system.

Experimental Example 2: Evaluation of biomarker sensing ability in urine sample of DGFET biosensor

The biosensor of the present disclosure operates through the process of directly processing unprocessed urine into each sensing channel of the EG. When an antibody immobilized on a reaction part of the biosensor and an antigen present in a urine sample are bound, a change in the surface potential of the biosensor is induced, and such a change is monitored by the shift of the bottom gate voltage at the reference current (1 nA) (see FIG. 3A).

In the case of the human blood, the blood had constant pH (pH 7.35-7.45). However, the pH of urine varies significantly from person to person (pH 4.5-8.0), which may affect the sensing characteristics. Therefore, to evaluate whether or not the biosensor of the present disclosure can stably sense biomarker in the urine sample, the pH stability of the DGFET was tested by using five consecutive cycles of pH 4, 6 (general normal), and 10. As a result of monitoring the bottom gate voltages at the reference current during the five cycles of pH change in the same EG channel, the voltage shift showed small hysteresis only (changes of 0.13 V, 0.095 V, and 0.029 V for pH 4, 6, and 10, respectively), providing that the sensor performance was stable in the urine sample reaction environment required in the present disclosure (see FIG. 3B). In addition, the small voltage shifts for the different pH solutions were shown (see FIG. 3C) (0.002 V, 0.021 V, and 0.007 V for pH 4, 6, 10, respectively) for 24 minutes, providing that the urine sample was used stably.

Experimental Example 3: Selection of experimental group

Urine samples obtained from normal people and PCa patients were divided into three groups: a normal group, a pre-DRE (digital rectal examination) PCa group, and a post-DRE PCa group. Meanwhile, benign prostatic hyperplasia (BPH) is similar to PCa in terms of causing enlargement of the prostate. However, the levels of specific biomarkers may be affected in BPH. Considering that BPH is a common disease in men over the age of 40, the normal group included urine samples from patients with BPH in the present disclosure to screen PCa accurately.

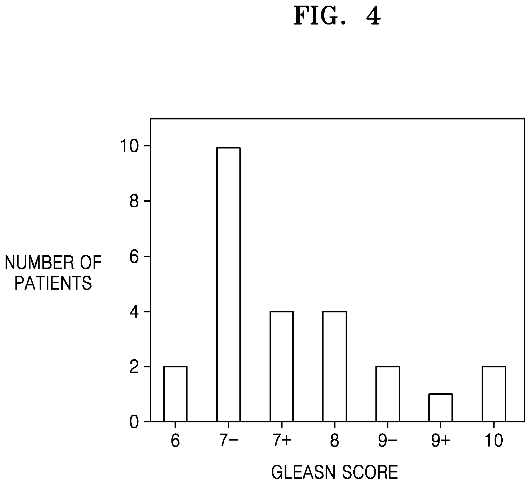

In the case of the normal group, the naturally voided urine samples were collected from the normal group during a regular health checkup. In the case of the patients with PCa, urine was collected twice, before and after the DRE procedure. The Gleason score of the sample population was shown in FIG. 4, and a summary of the information of 51 patients gathered/studied was provided in Table 1.

[Table 1]

|  | Normal | Cancer patient | P value |
|---|---|---|---|
| Number of subject | 26 | 25 | - |
| Median age (range) | 55.5 (31-79) | 66.1 (51-77) | <0.0001 |
| Median serum PSA (range), ng/ml | 1.2 (0.2-5.1) | 9.4 (3.4-44.2) | <0.0001 |
| Median prostate volume (range), ml | 31.5 (19-53) | 37.3 (14-106) | 0.2862 |

The differences in age and serum PSA levels between the normal group and the PCa group were statistically significant, whereas the differences in the prostate volume was not significant. Regarding unbiased prediction, none of these factors were considered in the ML algorithm.

Experimental Example 4: Evaluation of prostate cancer diagnosis using single-biomarker analysis

4-1: Analysis of DGFET biosensor sensing results using single-biomarker

For 76 urine samples obtained from Examples above, single-biomarker analysis was performed by using the DGFET biosensor.

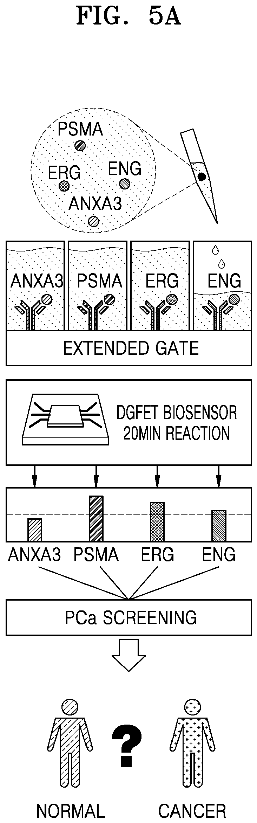

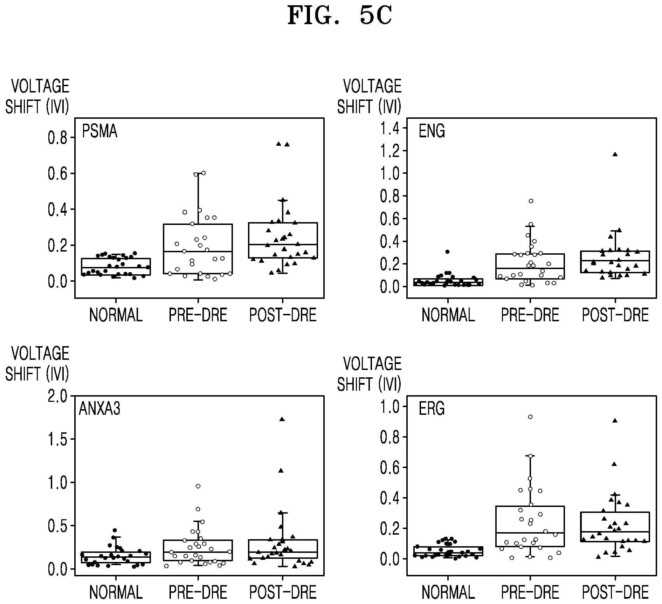

In detail, each urine sample was treated on four sensing channels, which were each conjugated with four antibodies capable of sensing four biomarkers. After 20 minutes, the biosensor measured the gate voltage shift at the reference current (1 nA). Each sample was measured three times, and a cutoff voltage value for the PCa screening was set from the shift voltage value induced by the normal pooled urine (0.19 V) (see FIG. 5A).

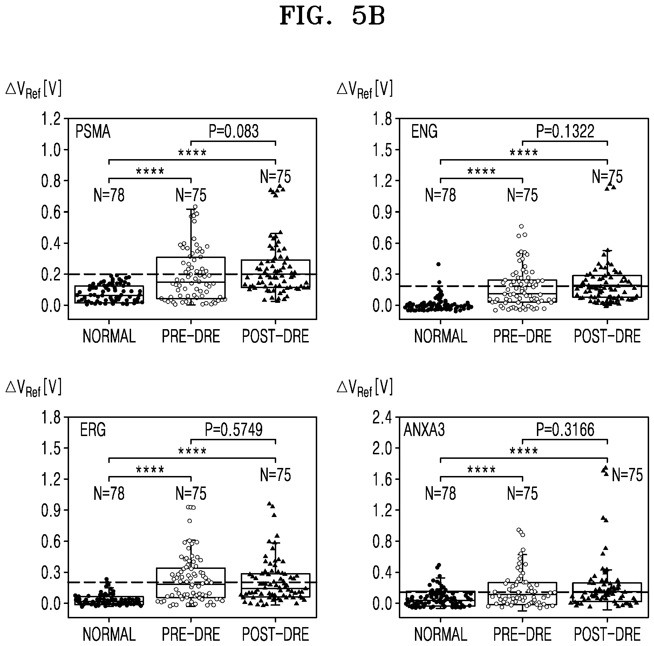

As a result of the analysis, in the case of PSMA, the pre-DRE urine sample and the post-DRE urine sample showed similar signals, but were distinguished from the normal group (see FIG. 5B). In detail, in the case of the normal group, only one of 78 data points reached close to the cut-off voltage shift, whereas, in the case of the cancer groups (pre-DRE and post-DRE), 30 and 38 data points of the 75 total data points exceeded the cut-off value, respectively. The p-values between the normal group and the cancer group with either pre-DRE or the post-DRE were less than 0.0001, suggesting that the signals were significantly distinguishable. However, the p-values between the cancer group with pre-DRE and the cancer group with post-DRE were calculated as 0.083, indicating that the two datasets were not statistically significant. Therefore, according to the sensing results obtained by using the PSMA single biomarker, it was confirmed that, when urine was directly sensed by using the biosensor of the present disclosure, the patients with PCa were screened at a similar level of accuracy, regardless of the DRE procedure. In addition, similar results were obtained when the ENG biomarker or the ERG biomarker were used.

However, the ANXA3 biomarker showed different tendency from the three biomarkers above. In detail, in ANXA3, the numbers of data points that exceeded the cut-off value in the pre- and post-DRE groups were similar to those for the other biomarkers (34 and 35 for the pre-DRE group and the post-DRE group, respectively). A significantly large proportion of the data points in the normal group had a larger voltage shift than the cut-off value (18 out of 78). Therefore, it was confirmed that the ANXA1 biomarker as a single-biomarker had the lowest sensing performance.

4-2: Evaluation of sensitivity, specificity, and accuracy of single biomarker

The sensitivity, specificity, and accuracy for the four biomarkers were analyzed.

The sensitivity is a value representing ability to correctly find a patient with a disease, and the specificity is a value representing ability to correctly find a normal person. That is, the sensitivity is a ratio at which a person with a disease is diagnosed as a disease, and the specificity is a ratio at which a normal person is diagnosed as a normal. The sensitivity and the specificity can be derived by the following formulae:

$$\text{Sensitivity} = TP / (TP + FN)$$

$$\text{Specificity} = TN / (TN + FP)$$

(True Positive (TP): a case of being diagnosed as positive (disease) by a diagnostic method in the model and also being correctly positive in reality;
True Negative (TN): a case of being diagnosed as negative (disease) by a diagnostic method in the model, and also being correctly negative in reality;
False Positive (FP): a case of being diagnosed as positive (disease) by a diagnostic method in the model, but being negative in reality; and
False Negative (FN): a case of being diagnosed as negative (disease) by a diagnostic method in the model, but being positive in reality.

In addition, the accuracy is a value representing ability to correctly find a person with a disease and a normal person, and can be derived from the following formula:

...

$$Accuracy = (TP + TN) / (TP + FN + TN + FP)$$

As a result of the analysis, the sensitivity was in a range of 43.3 % to 52.0 % in all biomarkers, wherein the ANXA3 biomarker had the lowest sensitivity. Regarding the specificity, PSMA, ERG, and ENG each had specificity of 94.9 % or more, whereas ANXA3 had specificity of only 76.9 %. Such low sensing performance of ANXA3 was similar to the box plot results of FIG. 5B in which sensing signals from all three experimental groups largely overlapped. In addition, regarding the accuracy, PSMA, ERG, and ENG each had similar accuracy (63.6 % to 66.7 %), whereas ANXA3 had accuracy of only 54.8 %.

Overall, it was confirmed that the screening performance was similar among the PSMA, ERG, and ENG biomarkers, whereas the screening performance of the ANXA3 biomarker was behind the other biomarkers. Considering the main purpose of the present disclosure, it is the most important to reduce the rate of false positive results, because the reduction can lead to a reduction in the number of unnecessary biopsies. In this regard, all four biomarkers outperform the PSA tests. However, the high specificity may lower the sensitivity. In the experimental results, the high level of specificity was resulted, but about half of the patients with PCa were missed. That is, the current single biomarker test can minimize unnecessary biopsies, but due to a high rate of false negatives, a significant portion of the patients with PCa may be missed. Therefore, there is a need to establish a strategy to increase the specificity without damaging the sensitivity.

4-3: Association analysis among biomarkers

To improve specificity in a way of using a multimarker, selection of biomarkers with low correlation is important. As the correlation among biomarkers is low, specificity may be improved. Thus, the correlation among the four biomarkers was analyzed.

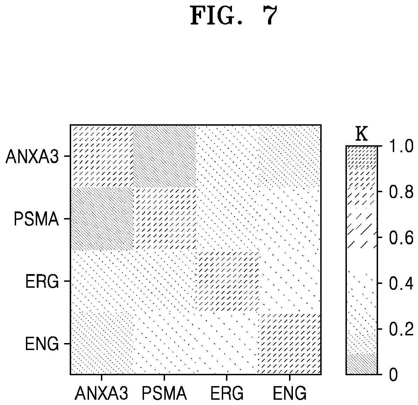

As a result, it was confirmed that, based on the Pearson correlation coefficient matrix of biomarker pairs consisting the four biomarkers, the ERG-ENG pair showed the highest correlation (k=0.27), whereas the ANXA3-PSMA pair showed the lowest correlation (k=0.04) (see FIG. 7). In addition, the average correlation coefficient of the individual biomarkers as 0.13, 0.15, 0.22, and 0.22 for ANXA3, PSMA, ERG, and ENG, respectively.

Accordingly, it was confirmed that the low $\kappa$ values for all pairs of the biomarkers indicate that these biomarkers were not correlated and showed remarkable sensing results. Therefore, it was concluded that the sensing performance can be significantly improved when the sensing signals obtianed from the four selected biomarkers are properly blended.

Experimental Example 5: Analysis of ML-based multimarker analysis

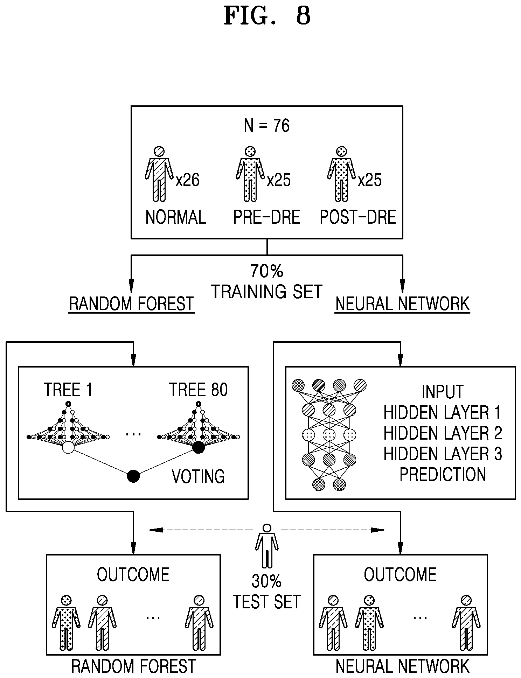

To confirm the importance of approach using the multimarker in diagnosing prostate, two general ML algorithms, RF (random forest) and NN (neural network) were applied. In detail, both algorithms were trained by 70 % of the total data set under supervised learning, and validated performance thereof by screening PCa from a blinded test set (30 % of the total data set) (see FIG. 8). The reference voltage shift of each biomarker was input into both algorithms to provide two different types of outputs, i.e., normal and Pca. Here, the cutoff value (0.19 V) was not considered in the algorithm. That is, both algorithms learn the raw data of the DGFET to consider even minor characteristics caused by urine that is not expected to have a significant impact.

5-1: Evaluation of relative importance of biomarker

The RF algorithm is useful in terms of understanding variables. For example, RF may be able to provide useful information of each biomarker in a decision-making process. In detail, the relative importance of each biomarker in the four-biomarker combination system was confirmed by using RF.

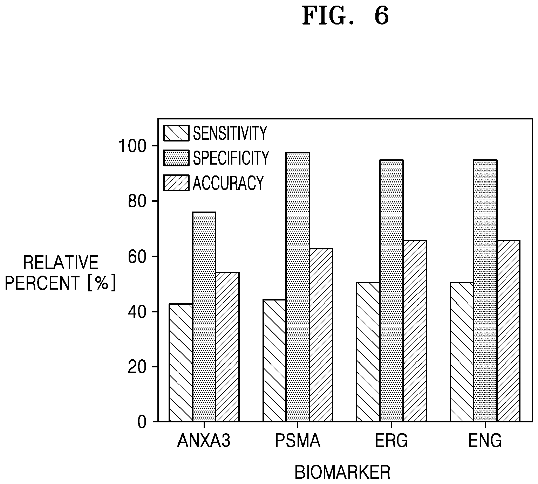

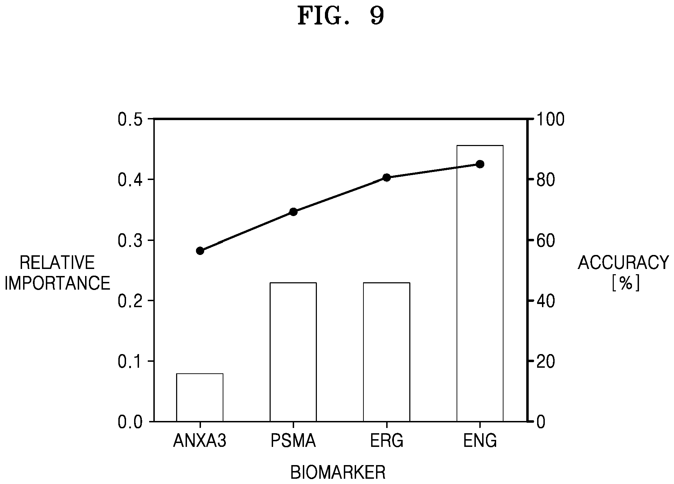

As a result, the PSMA, ERG, and ENG biomarkers that showed similar sensing performance in Examples above exhibited significantly different importance in the RF algorithm (see FIG. 9). PSMA and ERG showed the relative importance at a similar level (0.23 for both biomarkers), but ERG was found to have higher accuracy than PSMA, indicating that the relative importance does not necessarily mean the accuracy. In addition, in a similar manner as in the results of FIG. 6, ANXA3 showed the lowest importance (7 % only), suggesting that some biomarkers barely contribute to the diagnostic performance.

5-2: Evaluation of accuracy according to the number of biomarkers

When different numbers of biomarkers were used at the biomarker panel level, the average accuracy of PCa screening based on the RF and NN algorithms was confirmed.

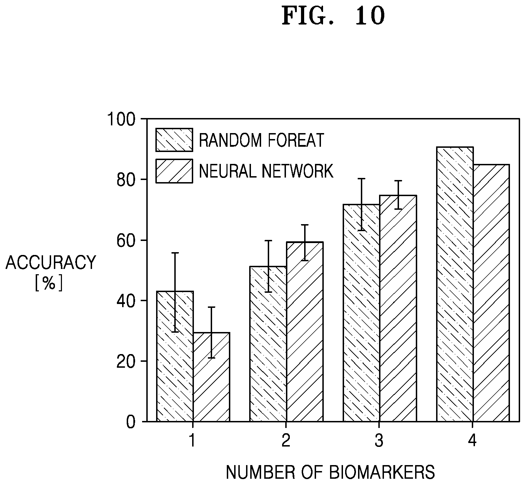

As a result, on average, the accuracy increased in both algorithms as the number of biomarkers in the panel increased (see FIG. 10). In detail, the accuracy of the RF and NN algorithms using a single biomarker was 73.2 % and 66.3 %, respectively. As the number of biomarkers increased, the average accuracy remarkably improved to 97.1 % in RF and 94.2 % in NN. Based on these results, referring to the improved average accuracy with increasing number of biomarkers in two different ML algorithms, it was confirmed that the multimarker approach provided more accurate clinical results.

5-3: Evaluation of PCa screening using multimarker - Predictor value

In general, the screening using ML is based on a predictor value, wherein the closer the predictor value is to either 0 or 1, the higher the certainty of the algorithm's prediction has. Thus, by using a combination of the four biomarkers, the predictor values of each biomarker panel were analyzed.

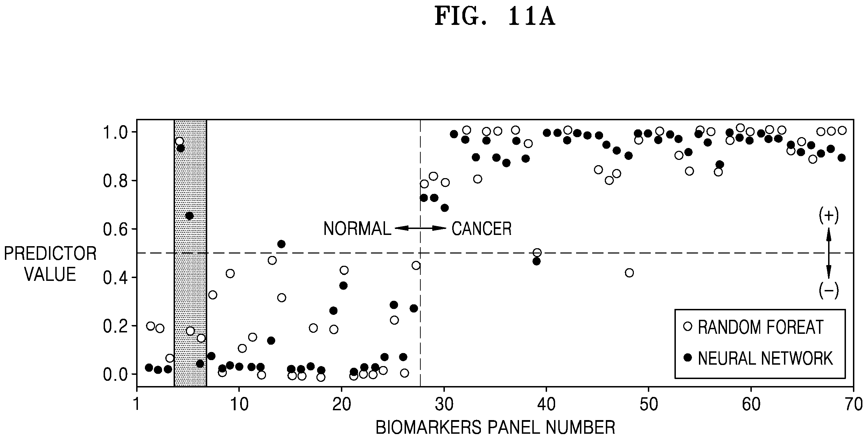

As a result, it was confirmed that, in terms of the biomarker panels, the RF algorithm was correct in all panels, except for one in each of the normal and biomarker panels. The NN algorithm missed three biomarker panels of the normal group and one biomarker panel of the cancer group (see FIG. 11A). As each urine sample was measured three times, the tree consecutive panel numbers correspond to each sample. For NN, two consecutive incorrect panels were found in one sample (panel numbers 4 and 5) (highlighted in gray).

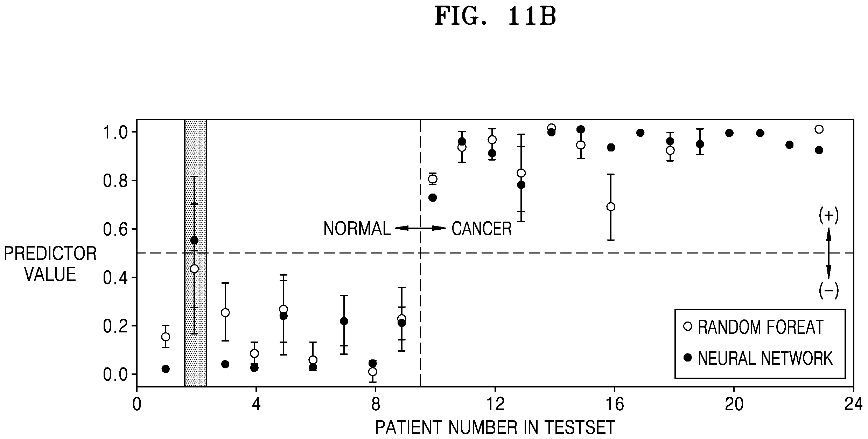

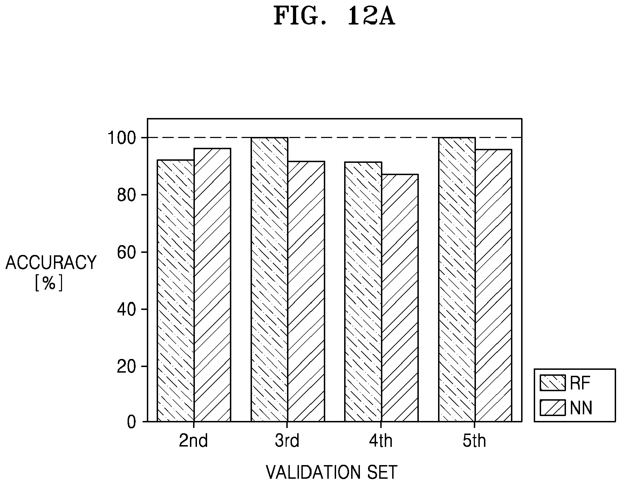

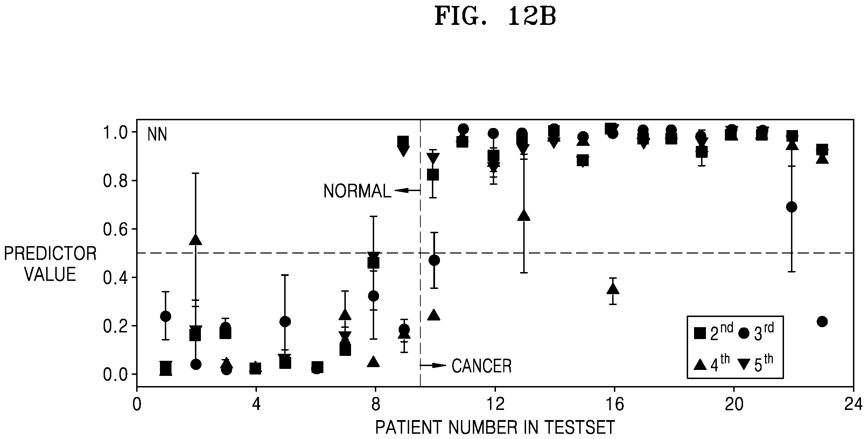

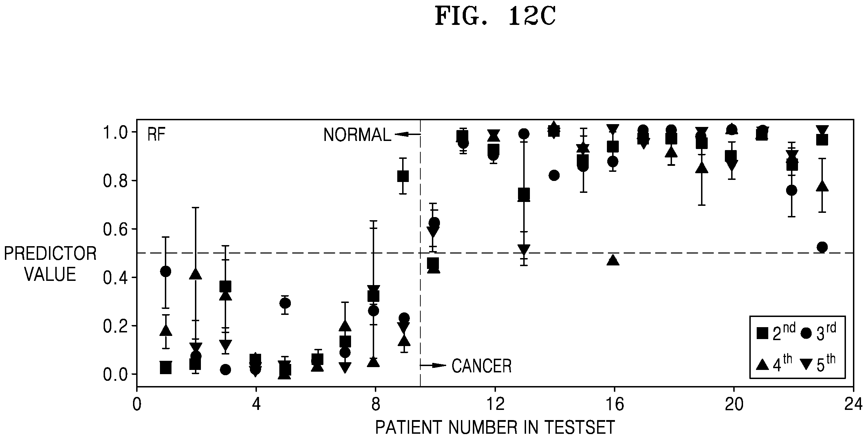

Next, based on the predictor value at the biomarker panel level, the average predictor value of each sample was analyzed. As a result, the accuracy of the RF algorithm was 100 %, meaning that there were no false results in the PCa screening. However, there were also samples with the predictor values close to 0.5. In the case of the NN algorithm, one false positive sample was found (highlighted in gray) (see FIG. 11B). Therefore, it is important to set an uncertainty part with respect to the algorithm analysis results, and patients belonging to this part are considered to take additional examination. Also, to validate analysis results in the limited number of data sets, four more predictions with different validation sets were performed and showed less than 5 % accuracy variations, confirming excellent performance of the screening (see FIGS.12A to 12C).

5-4: Evaluation of PCa screening using multimarker - Receiver operating characteristic (ROC) curve

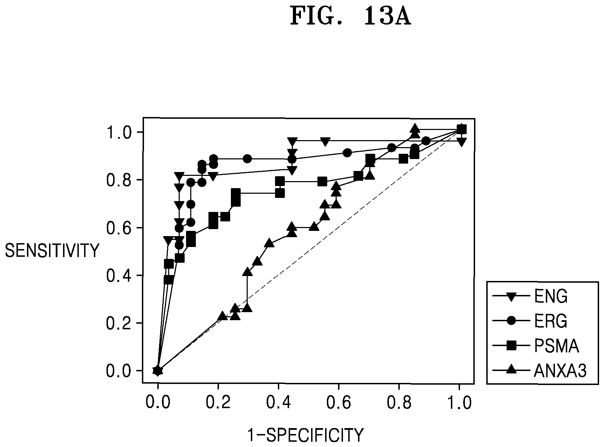

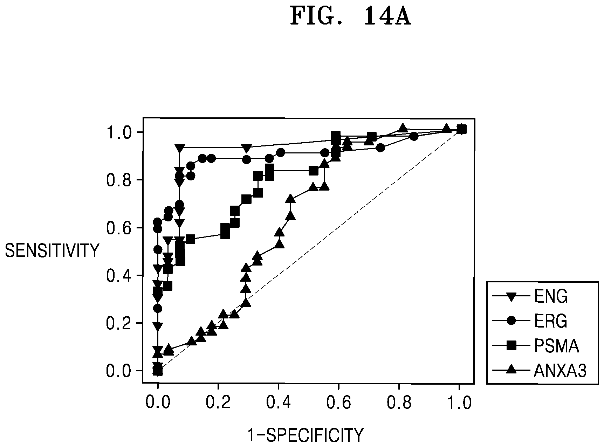

The performance of the ML-based screening model was analyzed by using ROC curves. In detail, ROC curves of the biomarker panel predicted by the RF algorithm with various numbers and combinations of biomarkers were analyzed. As a result, as the number of biomarkers increased, the ROC curves approached close to each axis, meaning an increase in accuracy (see FIGS. 13A to 13D). In addition, as the number of biomarkers increased, variations in the ROC curves decreased. Such a trend was also observed in the NN algorithm (see FIGS. 14A to 14D).

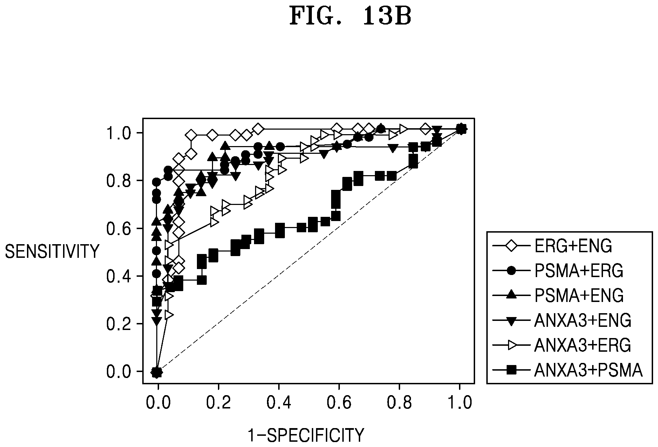

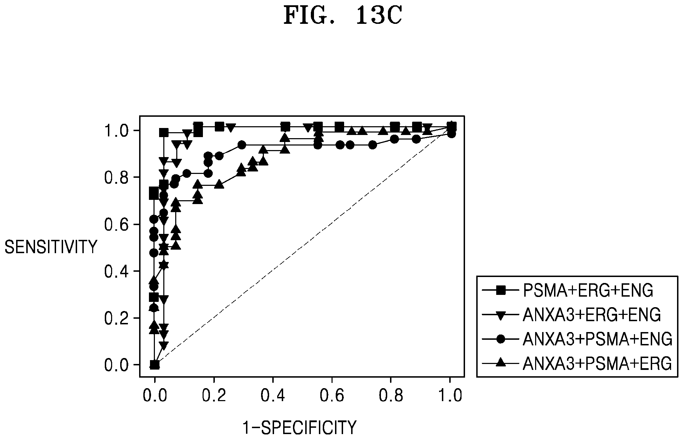

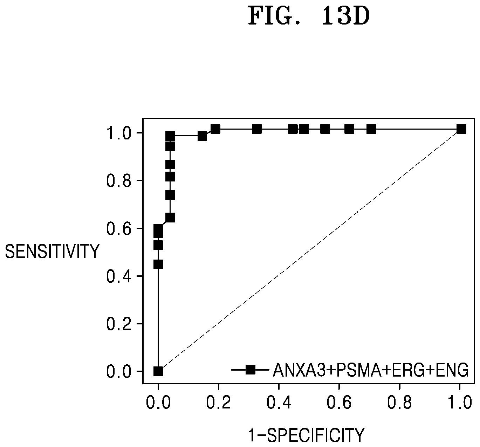

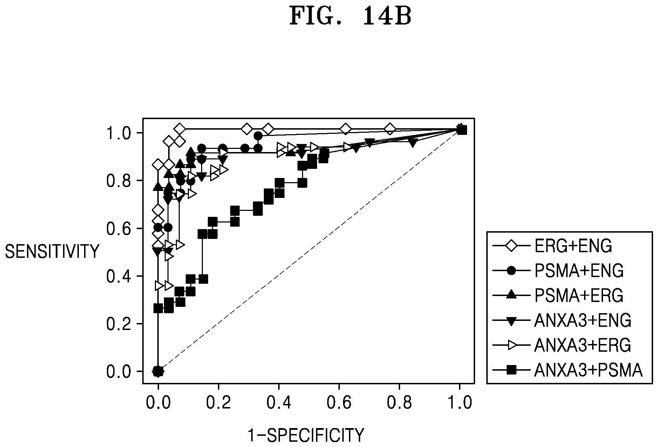

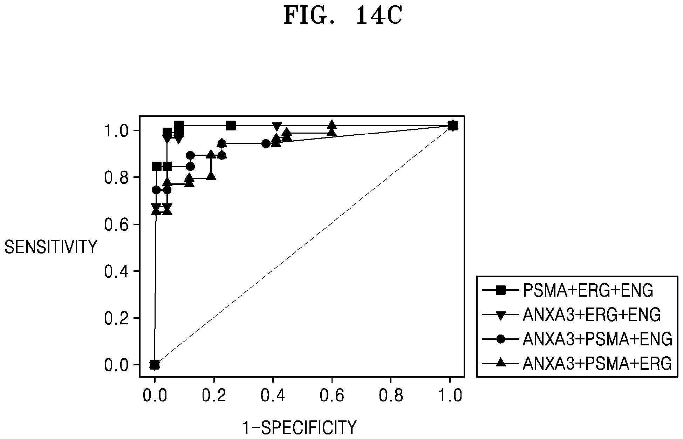

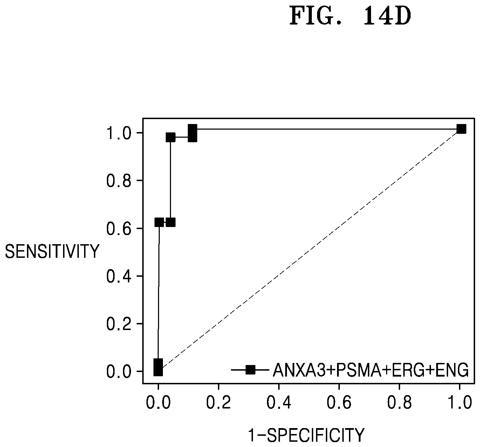

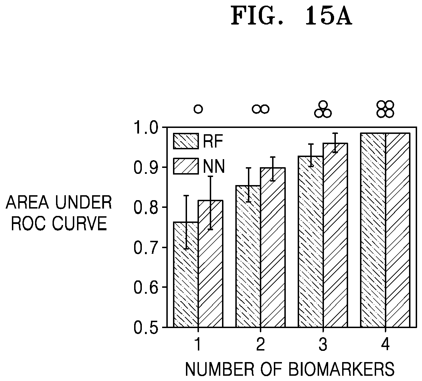

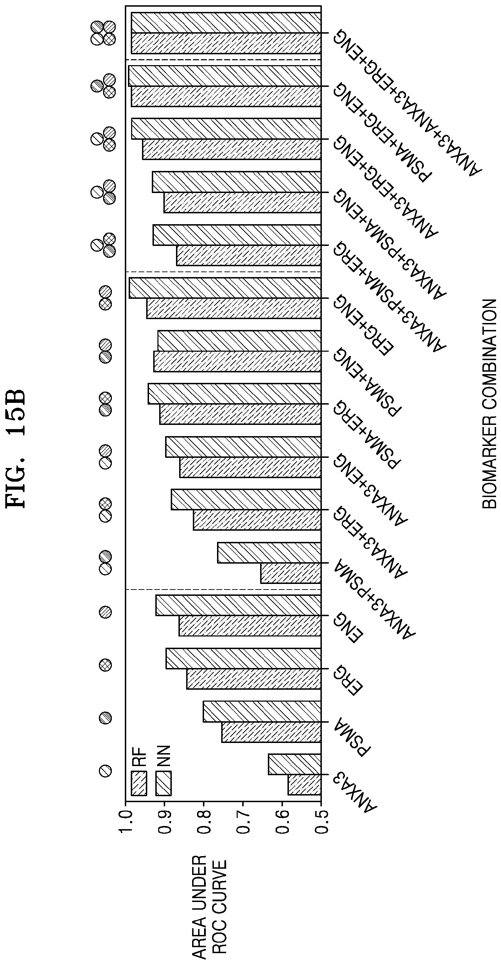

Next, area under ROC curve (AUROC) which is a single parameter representing the sensing performance of the ML model was analyzed. As a results of analyzing the AUROC with respect to the ROCs of FIGS. 13 and 14, it was confirmed that an AUROC value also increased as the number of biomarkers increased (see FIG. 15A). In detail, when a single biomarker was used, the AUROC value was the largest with ENG, followed by ERG, PSMA, and ANXA3. In addition, the AUROC value was significantly changed depending on a combination of biomarkers, and in this regard, the combination was analyzed to be more important than the number of biomarkers. For example, ENG + ERG had better sensing performance than PSMA + ERG + ANXA3 (see FIG. 15B). It was also confirmed that the inclusion of ANXA3 resulted in degraded sensing performance in both the RN and NN algorithms, and thus it can be seen that there is an optimal biomarker combination. For example, in consideration of the AUROC value, the optimal sensing results were achieved by ENG + ERG + PSMA in RF and ENG + ERG in NN.

Based on these results, it was confirmed that, regarding RF and NN that are complicated algorithms with nonlinear functions, simply increasing the number of biomarkers does not necessarily guarantee an increase in screening accuracy, and that the combination of biomarkers is also important.

Experimental Example 6: Clinical analysis of false signals

When analyzing patients with false signals in the biomarker-based prostate cancer screening system, the correlation between the ML prediction and the disease state of the patient was found, which can be used to improve the ML algorithm. In addition, such information can be used to construct a feedback loop for the patients. Accordingly, patients with high false rates can take additional tests to ensure that they do not have PCa. Therefore, first, when the test was identified with a different number of biomarkers, the RF-predicted average false rate of each biomarker panel was confirmed.

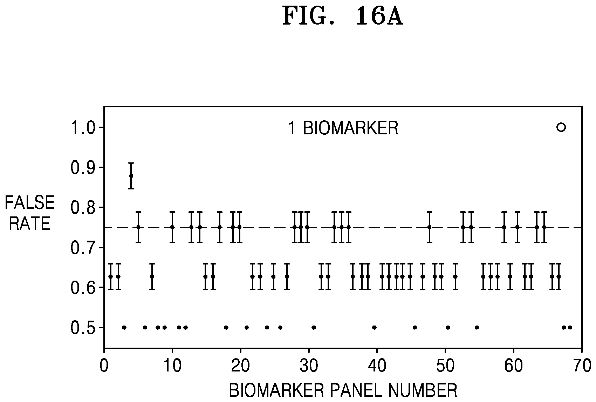

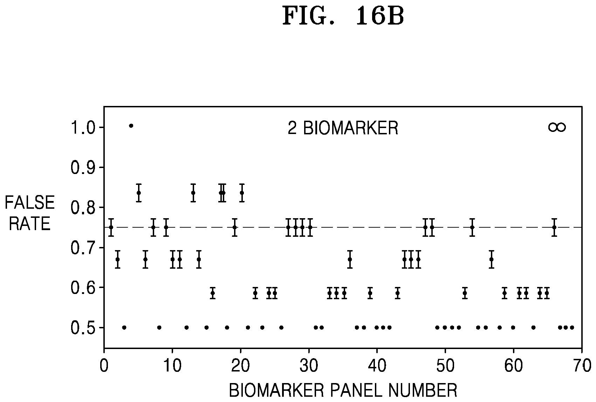

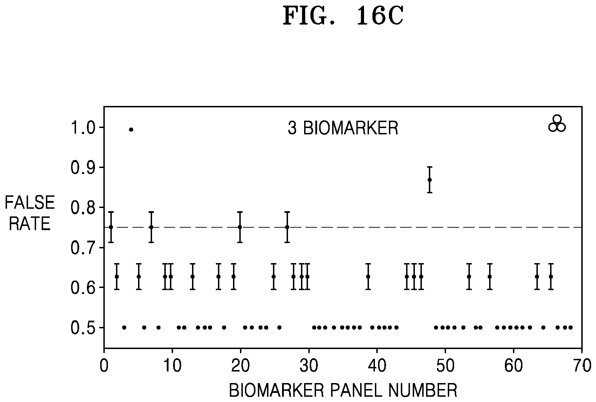

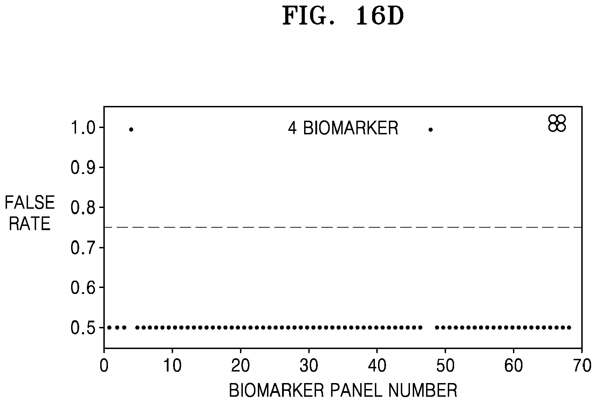

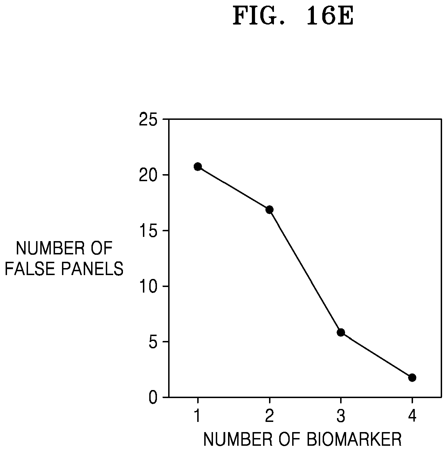

As a result, it was confirmed that the false rate decreased as the number of biomarkers increased. For example, when a combination of four biomarkers was used, only two false panels (see FIGS. 16A to 16E). However, the two false panels found always had false regardless of the number of biomarkers. In addition, as the number of biomarkers increased, the number of false panels having a false rate of higher than 0.5 gradually decreased (gradually decreased to 21, 17, 6, and 2 false panels).

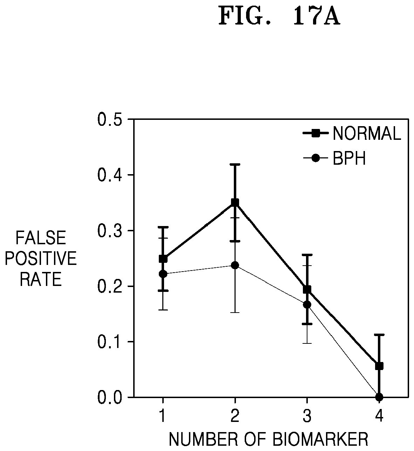

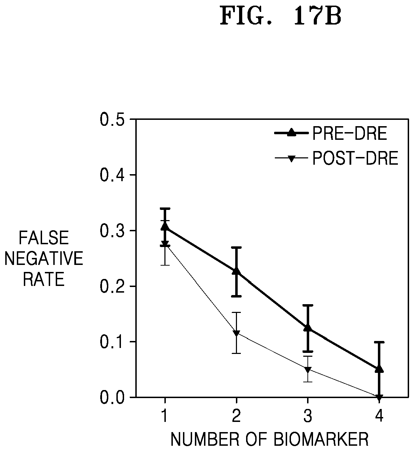

Next, as a result of confirming the false positive rates of the normal group and the BPH group by using various numbers of biomarkers, it was shown that, except for a combination of two biomarkers, there was no significant difference between the normal group and the BPH group (see FIG. 17A, p=0.03). In addition, as a result of confirming the false negative rates of the pre-DRE group and the post-DRE group by using various numbers of biomarkers, a combination of four biomarkers showed high sensitivity of 95.2 % or more in both groups. However, it was confirmed that the false negative rate was generally higher in the pre-DRE group (see FIG. 17b), indicating that the urine sample of the post-DRE group had better prostate cancer specificity.

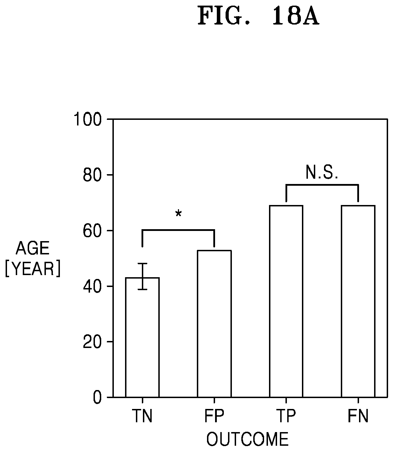

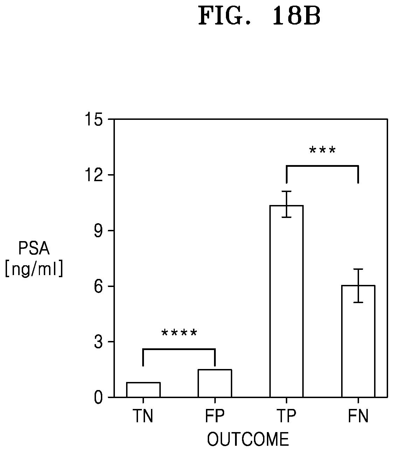

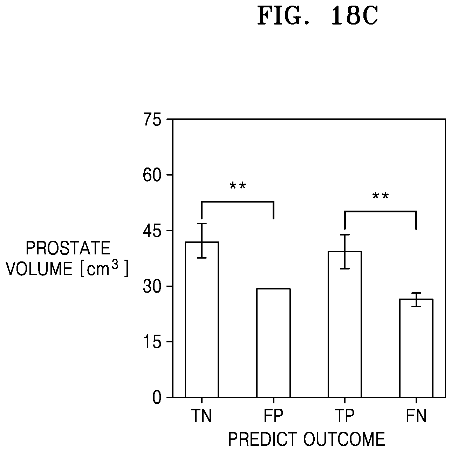

Next, since clinical parameters were not considered in the ML algorithm, clinical analysis of false matrix was performed to identify factors affecting false signals in the ML algorithm. As a result, in the normal group, elderly people were found to have a higher possibility of being incorrectly screened for PCa than younger people, but there was no statistical significance between the elderly people and the younger people in the cancer groups (see FIG. 18A). In contrast, people with high serum PSA levels were more likely to test positive in both the normal group and the cancer groups (see FIG. 18B). In addition, people with a low prostate volume were more likely to receive a false screening result in both the normal group and the cancer groups (see FIG. 18C). Therefore, it was confirmed that more accurate results can be obtained by considering clinical parameters in the prostate cancer screening system using the multimarker of the present disclosure.

The foregoing descriptions are only for illustrating the present disclosure, and it will be apparent to a person having ordinary skill in the art to which the present invention pertains that the embodiments disclosed herein can be easily modified into other specific forms without changing the technical spirit or essential features. Therefore, it should be understood that Examples described herein are illustrative in all respects and are not limited.

**Claims**

1. A biomarker composition for diagnosis of prostate cancer, comprising one or more selected from the group consisting of ANXA3 (annexin A3), PSMA (prostate-specific membrane antigen), ERG (erythroblast transformation-specific related gene protein), and ENG (endoglin).

2. The biomarker composition of claim 1, wherein the composition comprises ERG and ENG.

3. A composition for diagnosis of prostate cancer, comprising an agent(s) capable of measuring an expression level of one or more proteins selected from the group consisting of ANXA3 (annexin A3), PSMA (prostate-specific membrane antigen), ERG (erythroblast transformation-specific related gene protein), and ENG (endoglin), or genes encoding the same.

4. The composition of claim 3, wherein the composition comprises an agent(s) capable of measuring an expression level of ERG and ENG proteins or genes encoding the proteins.

5. A kit for diagnosis of prostate cancer, comprising the composition of claim 3 or 4.

6. The kit of claim 5, wherein the kit is an electrochemical biosensor.

7. A method of providing information on prostate cancer diagnosis, the method comprising measuring, in a biological sample isolated from a subject, an expression level of one or more proteins selected from the group consisting of ANXA3 (annexin A3), PSMA (prostate-specific membrane antigen), ERG (erythroblast transformation-specific related gene protein), and ENG (endoglin), or genes encoding the same.

8. The method of claim 7, further comprising:

   measuring, in a biological sample isolated from a control group, an expression level of one or more proteins selected from the group consisting of ANXA3, PSMA, ERG, and ENG, and genes encoding the same; and
   comparing the expression levels of the subject and the control group.

9. The method of claim 8, further comprising, when the expression level of the subject is higher than the control group, determining the subject as having developed prostate cancer or predicting risk of developing prostate cancer at a high level.

10. The method of claim 7, further comprising applying the measured expression level of the proteins or the genes encoding the same to a machine learning algorithm model.

11. The method of claim 10, wherein the machine learning algorithm model is learned by setting, as input values, 1) an expression level of one or more proteins selected from the group consisting of ANXA3, PSMA, ERG, and ENG, or genes encoding the same, in a prostate cancer patient and 2) an expression level of one or more proteins selected from the group consisting of ANXA3, PSMA, ERG, and ENG, and genes encoding the same, in a control group.

12. The method of claim 10, wherein the applying to the machine learning algorithm model comprises inputting the expression level of the proteins or the genes encoding the same measured in the subject to the machine learning algorithm model to output, as an output value, whether the subject has developed prostate cancer or is at risk of developing prostate cancer.

# FIG. 1A

# FIG. 1B

FIG. 2A

# FIG. 2B

FIG. 2C

FIG. 2D

# FIG. 3A

URINE

EXTENDED GATE

SURFACE POTENTIAL CHANGE

FET BIOSENSOR

FET

$V_G$ sweep

ELECTRICAL SIGNAL

① $V_{ref,1}$

② $V_{ref,2}$

DATA ANALYSIS

$V_{ref,1} < V_{ref,2}$

$I_{sd}$

$\triangle V_{ref}$

$V_G$

# FIG. 3B

# FIG. 3C

FIG. 4

# FIG. 5A

# FIG. 5B

# FIG. 5C

FIG. 6

FIG. 7

# FIG. 8

FIG. 9

# FIG. 10

# FIG. 11A

FIG. 11B

# FIG. 12A

FIG. 12B

FIG. 12C

FIG. 13A

FIG. 13B

FIG. 13C

FIG. 13D

FIG. 14A

FIG. 14B

# FIG. 14C

FIG. 14D

## FIG. 15A

FIG. 15B

## FIG. 16A

FIG. 16B

# FIG. 16C

FIG. 16D

# FIG. 16E

# FIG. 17A

# FIG. 17B

# FIG. 18A

# FIG. 18B

# FIG. 18C

## INTERNATIONAL SEARCH REPORT

| International application No. |
|---|
| **PCT/KR2021/017030** |

| **A.** | **CLASSIFICATION OF SUBJECT MATTER** |
|---|---|

**G01N 33/574**(2006.01)i; **G16B 15/30**(2019.01)i; **G16B 40/20**(2019.01)i; **G06N 3/08**(2006.01)i

According to International Patent Classification (IPC) or to both national classification and IPC

| **B.** | **FIELDS SEARCHED** |
|---|---|

Minimum documentation searched (classification system followed by classification symbols)

G01N 33/574(2006.01); A61K 39/395(2006.01); A61K 49/00(2006.01); C07K 16/32(2006.01); C07K 7/06(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 전립선암(prostate cancer), 바이오마커(biomarker), ANXA3, PSMA, ERG, ENG

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| X <br> Y | FUJITA, K. et al. Endoglin (CD105) as a urinary and serum marker of prostate cancer. Int. J. Cancer. 2009, vol. 124, pp. 664-669. <br> See abstract; page 665, left column and page 668, right column; and Conclusion. | 1,3,5-12 <br> 2,4 |
| Y | US 2011-0135643 A1 (SANDA, M. G. et al.) 09 June 2011 (2011-06-09) <br> See claims 196-197 and 199-203. | 2,4 |
| Y | ADAMO, P et al. The oncogene ERG: a key factor in prostate cancer. Oncogene. 2016, vol. 35, pp. 403-414. <br> See abstract; and page 406, left column and page 408, right column. | 2,4 |
| X | US 2013-0315830 A1 (CORNELL UNIVERSITY) 28 November 2013 (2013-11-28) <br> See claims 1-7; and paragraphs [0003] and [0006]. | 1,3,5-12 |
| X | KR 10-2010-0015421 A (PROTEOSYS AG) 12 February 2010 (2010-02-12) <br> See claims 1-13 and 18-20; and paragraphs [0002], [0005] and [0023]. | 1,3,5-8,10-12 |

| ☐ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **02 March 2022** | **02 March 2022** |

| Name and mailing address of the ISA/KR | Authorized officer |
|---|---|
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

<table>
<tr><td>International application No.</td></tr>
<tr><td>**PCT/KR2021/017030**</td></tr>
</table>

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**
Information on patent family members

International application No.

**PCT/KR2021/017030**

| Patent document cited in search report | | | Publication date (day/month/year) | Patent family member(s) | | | Publication date (day/month/year) |
|---|---|---|---|---|---|---|---|
| US | 2011-0135643 | A1 | 09 June 2011 | CA | 2723143 | A1 | 05 November 2009 |
| | | | | EP | 2300040 | A2 | 30 March 2011 |
| | | | | JP | 2011-519866 | A | 14 July 2011 |
| | | | | US | 2013-0230547 | A1 | 05 September 2013 |
| | | | | US | 8455615 | B2 | 04 June 2013 |
| | | | | WO | 2009-135019 | A2 | 05 November 2009 |
| | | | | WO | 2009-135019 | A3 | 08 April 2010 |
| US | 2013-0315830 | A1 | 28 November 2013 | CA | 2865774 | A1 | 06 September 2013 |
| | | | | EP | 2819704 | A1 | 07 January 2015 |
| | | | | HK | 1202256 | A1 | 25 September 2015 |
| | | | | JP | 2015-508903 | A | 23 March 2015 |
| | | | | WO | 2013-130177 | A1 | 06 September 2013 |
| KR | 10-2010-0015421 | A | 12 February 2010 | AU | 2008-238258 | A1 | 23 October 2008 |
| | | | | AU | 2008-238258 | B2 | 23 May 2013 |
| | | | | BR | PI0810176 | A2 | 30 December 2014 |
| | | | | CA | 2682899 | A1 | 23 October 2008 |
| | | | | CN | 101669032 | A | 10 March 2010 |
| | | | | EP | 2135084 | A1 | 23 December 2009 |
| | | | | JP | 2010-523990 | A | 15 July 2010 |
| | | | | MX | 2009010910 | A | 20 January 2010 |
| | | | | RU | 2009141703 | A | 20 May 2011 |
| | | | | US | 2010-0129834 | A1 | 27 May 2010 |
| | | | | US | 8377648 | B2 | 19 February 2013 |
| | | | | WO | 2008-125262 | A1 | 23 October 2008 |

Form PCT/ISA/210 (patent family annex) (July 2019)

**EP 4 253 957 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Adv. Sci.,* 2019, vol. 6 (11), 1802115